(19) Europäisches Patentamt / European Patent Office / Office européen des brevets

(11) **EP 4 734 122 A1**

(12) **EUROPEAN PATENT APPLICATION**

(43) Date of publication:
29.04.2026 Bulletin 2026/18

(21) Application number: 24222915.1

(22) Date of filing: 23.12.2024

(51) International Patent Classification (IPC):
**G16H 20/40** (2018.01) **G16H 40/40** (2018.01)

(52) Cooperative Patent Classification (CPC):
(C-Sets available)
**A61M 15/0065; G16H 20/13; G16H 40/40;
G16H 40/63;** A61M 2016/0036; A61M 2202/0468;
A61M 2205/70; A61M 2230/40 (Cont.)

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: 28.10.2024 CN 202411515338
22.11.2024 CN 202411697776

(71) Applicant: Feellife Health Inc.
Shenzhen, Guangdong 518000 (CN)

(72) Inventors:
• HUA, Jian
Shenzhen, 518000 (CN)
• ZHENG, Baoda
Shenzhen, 518000 (CN)
• MOENS, Didier
Shenzhen, 518000 (CN)

(74) Representative: Redl, Gerda
Redl Life Science Patents OG
Donau-City-Straße 11
1220 Wien (AT)

(54) **NEBULIZATION CONTROL METHOD AND SYSTEM, AND ELECTRONIC DEVICE**

(57) A nebulization control method and system, and an electronic device are disclosed. The method includes: obtaining initial respiration data of a target user according to a collection time period; obtaining user respiration parameters by performing respiration parameter calculation for the target user according to the collection time period and the initial respiration data; obtaining a user respiration state by performing respiration state detection for the target user according to the user respiration parameters; and adjusting nebulization parameters for the nebulization device according to the user respiration state and the user respiration parameters.

Obtaining initial respiration data of a target user according to a preset collection time period — S101

Obtaining user respiration parameters by performing respiration parameter calculation for the target user according to the collection time period and the initial respiration data — S102

Obtaining a user respiration state by performing respiration state detection for the target user according to the user respiration parameters — S103

Adjusting nebulization parameters for the nebulization device according to the user respiration state and the user respiration parameters — S104

FIG. 1

(52) Cooperative Patent Classification (CPC): (Cont.)

C-Sets
A61M 2202/0468, A61M 2202/0007;
A61M 2230/40, A61M 2230/005

## Description

### TECHNICAL FIELD

The present disclosure relates to the technical field of medical devices, and in particular, to a nebulization control method and system, and an electronic device.

### BACKGROUND

Nebulization devices are medical devices designed to convert a drug solution into tiny aerosol particles, enabling the drug to be inhaled into the lungs through the patient's breathing, thereby treating respiratory diseases. However, conventional nebulization devices operate in a fixed mode, which limits their ability to intelligently adjust the nebulization time and nebulization rate. Therefore, the conventional nebulization devices have single operating mode and cannot be adjusted according to the user real-time respiration state. Therefore, the nebulization devices exhibit limited operating flexibility, resulting in inefficient utilization of nebulized drugs.

Therefore, it has become an urgent technical problem to be solved how to improve the operating flexibility of a nebulization device and thereby promote the utilization of nebulized drugs.

### SUMMARY

The main objective of the present disclosure is to propose a nebulization control method and system, and an electronic device, which aim to improve the operating flexibility of a nebulization device and thereby promote utilization of nebulized drugs.

In order to achieve the above objective, according to an embodiment in a first aspect of the present disclosure, a nebulization control method is provided, which is applied to a nebulization device. The method includes:

> obtaining initial respiration data of a target user according to a collection time period;
>
> obtaining user respiration parameters by performing respiration parameter calculation for the target user according to the collection time period and the initial respiration data;
>
> obtaining a user respiration state by performing respiration state detection for the target user according to the user respiration parameters; and
>
> adjusting nebulization parameters for the nebulization device according to the user respiration state and the user respiration parameters.

In some embodiments, the user respiration parameters comprise exhalation time interval information, inhalation depth data and inhalation time interval information; the nebulization parameters comprise a nebulization start-stop time and a nebulization rate; and adjusting nebulization parameters for the nebulization device according to the user respiration state and the user respiration parameters comprises:

> adjusting the nebulization start-stop time for the nebulization device according to the user respiration state, the exhalation time interval information, the inhalation depth data and the inhalation time interval information; and
>
> adjusting the nebulization rate for the nebulization device according to the user respiration state and the inhalation depth data.

In some embodiments, adjusting the nebulization start-stop time for the nebulization device according to the user respiration state, the exhalation time interval information, the inhalation depth data and the inhalation time interval information comprises:

> selecting stable time interval information from the exhalation time interval information and the inhalation time interval information according to the user respiration state; wherein the stable time interval information consists of the exhalation time interval information and the inhalation time interval information which are continuous, and the user respiration state comprises a stable respiration state;
>
> obtaining a total stable duration of the stable time interval information;
>
> in response to the total stable duration being greater than a preset stable duration threshold, selecting target time interval information from the stable time interval information according to the stable respiration state and the inhalation time interval information; and
>
> adjusting the nebulization start-stop time for the nebulization device according to the target time interval information.

In some embodiments, adjusting the nebulization start-stop time for the nebulization device according to the target time interval information comprises:

> using at least two consecutive pieces of target time interval information as historical time interval information, according to a preset time interval selection rule;
>
> obtaining a historical duration for each historical time interval information;

determining an offset duration according to a preset sensing outlet distance and the historical time interval information; wherein the offset duration indicates a duration required for the initial respiration data to pass through the sensing outlet distance, and the sensing outlet distance is a distance between a respiration sensing module and a drug administration route outlet;

determining offset time point information according to the offset duration and the historical duration; and

adjusting the nebulization start-stop time for the nebulization device according to the offset time point information.

In some embodiments, adjusting the nebulization rate for the nebulization device according to the user respiration state and the inhalation depth data comprises:

selecting a base rate from preset candidate rates according to the user respiration state;

selecting a target rate from the candidate rates according to a preset drug viscosity, the base rate and the inhalation depth data; and

adjusting the nebulization rate for the nebulization device according to the target rate.

In some embodiments, after obtaining initial respiration data of a target user according to a collection time period, the method further comprises:

determining gas flow rate data according to the collection time period and the initial respiration data;

selecting initial calibration data from the gas flow rate data according to a preset calibration data selection rule and the collection time period;

obtaining error evaluation information by performing error evaluation on the initial calibration data according to a preset differential calculation rule;

determining an accumulated duration according to the error evaluation information, the initial calibration data and the collection time period;

obtaining duration comparison information by comparing the accumulated duration and a preset calibration duration;

obtaining zero calibration detection information by performing zero calibration detection on the initial respiration data according to the duration comparison information;

determining zero calibration enabled/disabled state of the initial respiration data according to the zero calibration detection information; and

performing zero calibration on the initial respiration data according to the zero calibration enabled/disabled state.

In some embodiments, obtaining error evaluation information by performing error evaluation on the initial calibration data according to the preset differential calculation rule comprises:

obtaining first-order differential data and second-order differential data for the initial calibration data according to the differential calculation rule;

obtaining first comparison information by comparing the first-order differential data with a preset first threshold;

obtaining second comparison information by comparing the second-order differential data with a preset second threshold; and

obtaining the error evaluation information by comparing the first comparison information and the second comparison information with preset differential comparison information respectively.

In order to achieve the above objective, according to an embodiment in a second aspect of the present disclosure, a nebulization control system is proposed, for implementing the nebulization control method according to the embodiments in the first aspect. The system comprises a central processor, the central processor comprises:

a data acquisition unit, configured to acquire initial respiration data of a target user according to a collection time period;

a data processing unit, configured to obtain user respiration parameters by performing respiration parameter calculation for the target user according to the collection time period and the initial respiration data;

a state detection unit, configured to obtain a user respiration state by performing respiration state detection for the target user according to the user respiration parameters; and

a parameter adjustment unit, configured to adjust nebulization parameters of the nebulization device according to the user respiration state and the user respiration parameters.

In some embodiments, the system further comprises: a power supply module, a parameter setting module, a liquid nebulization module, a respiration sensing module and a prompt module, wherein,

an output end of the parameter setting module is electrically connected to an input end of the central processor, to set the nebulization parameters;

an output end of the respiration sensing module is electrically connected to the input end of the central processor, to collect the initial respiration data;

an output end of the power supply module is electrically connected to the input end of the central processor, to supply power to the central processor;

an input end of the liquid nebulization module is electrically connected to an output end of the central processor, and an output end of the liquid nebulization module is connected to an input end of a drug administration route, to perform drug nebulization according to the nebulization parameters; and

an input end of the prompt module is electrically connected to the output terminal of the central processor, to prompt the user respiration state according to prompt information.

In order to achieve the above objective, according to an embodiment in a second aspect of the present disclosure, an electronic device is proposed, including a memory and a processor, the memory stores a computer program which, when executed by the processor, implements the nebulization control method according to the embodiments in the first aspect.

The nebulization control method and system, and the electronic device are proposed in the present disclosure. Firstly, initial respiration data of a target user is obtained according to a preset collection time period, and respiration parameters of the target user are calculated according to the collection time period and the initial respiration data, to obtain user respiration parameters. Then, a respiration state of the target user is detected according to the user respiration parameters, to obtain a user respiration state. Finally, nebulization parameters of the nebulization device are adjusted according to the user respiration state and the user respiration parameters. Therefore, in the nebulization control method provided by embodiments of the present disclosure, the nebulization parameters of the nebulization device can be adjusted according to the real-time respiration data, so that the user can inhale the nebulized drugs in a specific respiration state, which improves the operating flexibility of the nebulization device, and also reduces the drug waste due to the drug nebulization for a long time, thus improving the utilization of nebulized drugs.

**BRIEF DESCRIPTION OF DRAWINGS**

FIG. 1 is an optional flow chart of a nebulization control method provided by an embodiment of the present disclosure;

FIG. 2 is another optional flow chart of the nebulization control method provided by an embodiment of the present disclosure;

FIG. 3 is a flow chart of step S203 in FIG. 2;

FIG. 4 is a flow chart of step S 104 in FIG. 1;

FIG. 5 is a flow chart of step S401 in FIG. 4;

FIG. 6 is a flow chart of step S504 in FIG. 5;

FIG. 7 is a flow chart of step S402 in FIG. 4;

FIG. 8 is a schematic structural view of a nebulization control system provided by an embodiment of the present disclosure;

FIG. 9 is a schematic structural view of a central processor provided by an embodiment of the present disclosure; and

FIG. 10 is a schematic view showing hardware structures of an electronic device provided by an embodiment of the present disclosure.

**DETAILED DESCRIPTION**

In order to make the objectives, technical solutions and advantages of the disclosure clearer, the disclosure will be further described in detail below with reference to the accompanying drawings and embodiments. It should be understood that the specific embodiments described here are only used to explain the disclosure and are not intended to limit the disclosure.

It should be noted that although the functional modules are divided in the device schematic diagram and the logical sequence is shown in the flow chart, in some cases, the steps shown or described may be performed differently from the module division in the device or performed in a different sequence in the flow chart. The terms such as "first", "second" in the description, the claims, and the above-mentioned accompanying drawings are used to distinguish similar objects, and not necessarily used to describe a specific order or sequence.

Unless otherwise defined, all technical and scientific terms used herein have the same meaning as commonly understood by those of ordinary skills in the art. The terms used herein are only for the purpose of describing embodiments of the present disclosure and are not intended to limit the present disclosure.

Firstly, several terms involved in the present disclosure are explained.

Artificial intelligence (AI) refers to a new technical science that studies and develops theories, methods, technologies and application systems for simulating, extending and expanding human intelligence. AI is a branch of computer science, which attempts to understand the essence of intelligence and produce a new intelligent machine that can respond in a manner similar to human intelligence. Research in this field includes robotics, language recognition, image recognition, natural language processing and expert systems, and the like. AI can simulate the information process of human consciousness and thought. It is also a theory, method, technology and application system that uses digital computers or machines controlled by digital computers to simulate, extend and expand human intelligence, perceive the environment, acquire knowledge and use that knowledge to obtain the optimal results.

Therefore, an embodiment of the present disclosure provides a nebulization control method and system, and an electronic device, which aims to improve the operating flexibility of the nebulization device and thereby promote the utilization of nebulized drugs.

The nebulization control method and system, and the electronic device provided by an embodiment of the present disclosure are specifically described through the following embodiments. First, the nebulization control method in an embodiment of the present disclosure is described.

In an embodiment of the present disclosure, relevant data may be obtained and processed, according to AI technologies. AI is a theory, method, technology and application system that uses digital computers or machines controlled by digital computers to simulate, extend and expand human intelligence, perceive the environment, acquire knowledge and use that knowledge to obtain the optimal results.

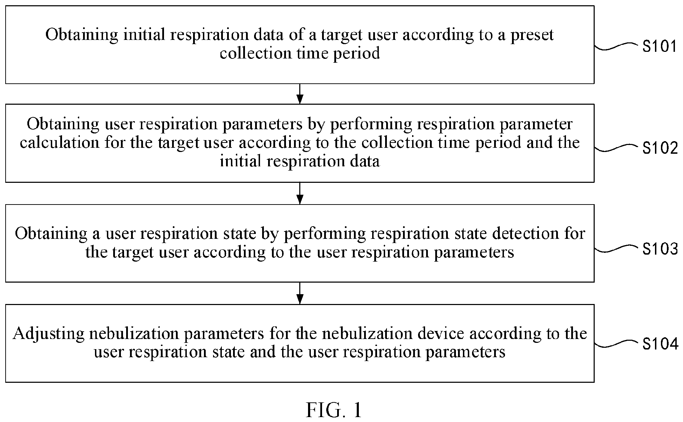

FIG. 1 is an optional flow chart of a nebulization control method provided by an embodiment of the present disclosure. The method in FIG. 1 is applied to a nebulization device. The nebulization control method may include but is not limited to steps S101 to S104:

step S101, obtaining initial respiration data of a target user according to a collection time period;

step S 102, obtaining user respiration parameters by performing respiration parameter calculation for the target user according to the collection time period and the initial respiration data;

step S103, obtaining a user respiration state, by performing respiration state detection for the target user according to the user respiration parameters; and

step S104, adjusting nebulization parameters for the nebulization device according to the user respiration state and the user respiration parameters.

In steps S101 to S104 illustrated in an embodiment of the present disclosure, firstly, initial respiration data of a target user is obtained according to a collection time period, and respiration parameters of the target user are calculated according to the collection time period and the initial respiration data to obtain user respiration parameters. Then, a respiration state of the target user is detected according to the user respiration parameters to obtain a user respiration state. Finally, nebulization parameters of the nebulization device are adjusted according to the user respiration state and the user respiration parameters. Therefore, according to the nebulization control method provided by an embodiment of the present disclosure, the nebulization parameters of the nebulization device can be adjusted according to the real-time respiration data, so that the user can inhale the nebulized drugs in a specific respiration state, which improves the operating flexibility of the nebulization device, and also reduces the drug waste due to the long duration of drug nebulization, thus improving the utilization of nebulized drugs.

In step S 101 of some embodiments, the collection time period may be a fixed value for describing the time interval of initial respiration data collection. It may be millisecond-level or microsecond-level. The specific value may be set according to the actual needs of those of ordinary skills in the art without specific limitation to the present disclosure. The initial respiration data may include, but is not limited to, at least one of: temperature data, humidity data and air pressure data. The initial respiration data is used to calculate parameters related to the respiration situation of the target user, which may be collected according to the sensor on the nebulization device.

In other embodiments, in step S 101, the collection time interval for the initial respiration data may also be determined according to a preset collection algorithm or a specific formula, instead of setting a fixed value for all collection time intervals of the initial respiration data to satisfy the needs of specific initial respiration data collection.

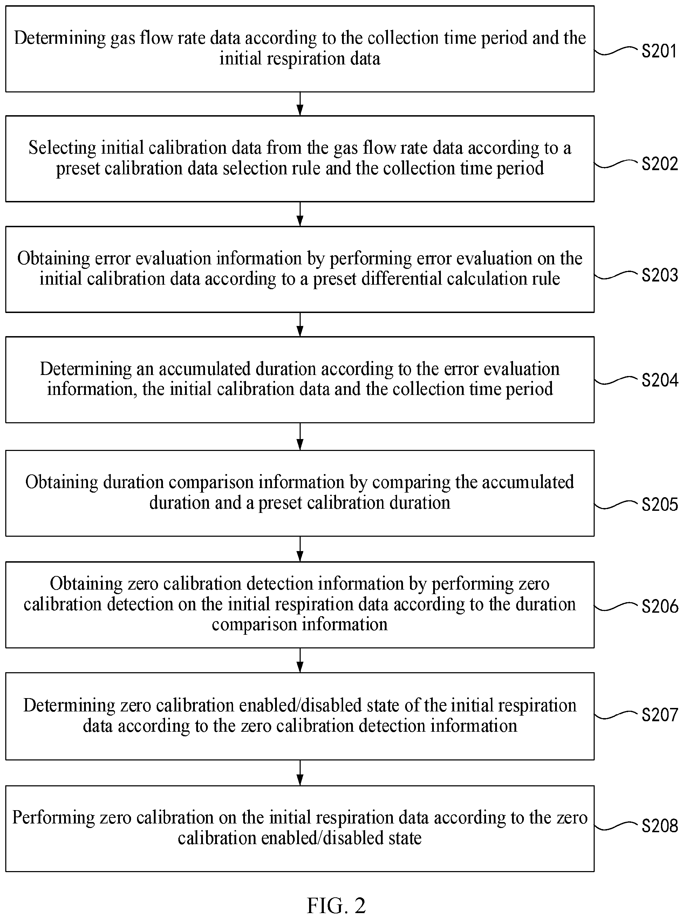

Referring to FIG. 2, in some embodiments, after step S101, the nebulization control method may also include, but is not limited to, steps S201 to S208:

step S201, determining gas flow rate data according to the collection time period and the initial respiration data;

step S202, selecting initial calibration data from the gas flow rate data according to a preset calibration data selection rule and the collection time period;

step S203, obtaining error evaluation information by performing error evaluation on the initial calibration data according to a preset differential calculation rule;

step S204, determining an accumulated duration, according to the error evaluation information, the initial calibration data and the collection time period;

step S205, obtaining duration comparison information by comparing the accumulated duration and a preset calibration duration;

step S206, obtaining zero calibration detection information by performing zero calibration detection on the initial respiration data according to the duration comparison information;

step S207, determining zero calibration enabled/disabled state of the initial respiration data according to the zero calibration detection information; and

step S208, performing zero calibration on the initial respiration data, according to the zero calibration enabled/disabled state.

In some embodiments, in step S201, the determining gas flow rate data according to the collection time period and the initial respiration data may be implemented as follows. Firstly, the initial respiration data is calibrated through a flow simulation sensor to obtain the calibrated respiration data. Then, the calibrated respiration data is used to fit the gas flow rate curve. Finally, the gas flow rate data is determined from the gas flow rate curve according to the collection time period.

In some embodiments, in step S202, the initial calibration data includes first calibration data and second calibration data. The preset calibration data selection rule is as follows: the last collection time period is selected as a target time period, the gas flow rate data corresponding to the target time period is used as the first calibration data, and the second calibration data is selected forward according to the first initial calibration data. The number of the second calibration data is at least three to satisfy the minimum number requirement of initial calibration data required by the differential calculation rule. When the accumulated duration is greater than or equal to the total number of four collection time periods, the number of the second calibration data is determined according to the accumulated duration and the collection time period. Specifically, first, the number of the collection time periods is determined according to the accumulated duration, which is regarded as the number of accumulated periods. Then, the continuous collection time periods are selected as historical durations, according to the target time period and the number of accumulated periods. The total number of the number of the historical durations and the number of the target time period is equal to the number of accumulated periods, and the historical durations and the target time period are continuous. Finally, the gas flow rate data corresponding to the historical durations is selected as the second calibration data.

In some embodiments, in step S204, the accumulated duration may be determined according to the error evaluation information, the initial calibration data, and the collection time period. Specifically, the error evaluation information is used to describe whether the initial calibration data passes the error evaluation. If the error evaluation information indicates that the initial calibration data passes the error evaluation, then the accumulated duration is determined according to the total number of the initial calibration data and the length of the collection time period. If the collection time period is a fixed value, then the accumulated duration may be obtained by multiplying the total number of the initial calibration data and the collection time period. If the value of each collection time period is not fixed, then the historical duration and the target time period are first determined, according to the initial calibration data, and the historical duration and the target time period are then added to obtain the accumulated duration. If the error evaluation information indicates that the initial calibration data cannot pass the error evaluation, then the accumulated duration is determined to be 0, and step S201 is re-executed to determine the next gas flow rate data, according to next collection time period and next initial respiration data.

In some embodiments, in S205, the calibration duration is used to describe the minimum accumulated duration required for zero calibration. The specific value of the calibration duration may be 6 or 10 seconds, or may be selected according to the actual needs by those of ordinary skills in the art.

In some embodiments, in step S206, the zero calibration detection information is used to describe whether or not the zero calibration of the initial respiration data can be performed. Since the gas flow rate data is obtained by fitting according to the calibrated respiration data, the initial respiration parameters corresponding to the initial calibration data may be determined according to the duration comparison information to perform zero calibration detection on the initial respiration data to obtain the zero calibration detection information. Specifically, if the duration comparison information indicates that the accumulated duration is greater than or equal to the calibration duration, which means that within the calibration duration, the initial calibration data obtained is sufficient to determine that the gas flow rate data has been stable to perform the zero calibration of the initial respiration data, then the zero calibration detection information is set to indicate that the zero calibration can be performed. If the duration comparison information indicates that the accumulated duration is less than the calibration duration, which means that within the accumulated duration, the initial calibration data obtained is not enough to determine whether the gas flow rate data is stable, and the zero calibration of the initial respiration data cannot be performed, then the zero calibration detection information is set to indicate that the zero calibration cannot be performed, and step S201 is executed again to determine the gas flow rate data according to the collection time period and the initial respiration data.

In some embodiments, in step S207, the zero calibration enabled/disabled state of the initial respiration data includes a calibration function enabled state and a calibration function disabled state. If the zero calibration detection information indicates that zero calibration can be performed, then the zero calibration enabled/disabled state of the initial respiration data is set to the calibration function enabled state. If the zero calibration detection information indicates that zero calibration cannot be performed, then the zero calibration enabled/disabled state of the initial respiration data is set to the calibration function disabled state.

In some embodiments, in step S208, if the zero calibration enabled/disabled state is the calibration function enabled state, then the zero calibration is performed on the initial respiration data. If the zero calibration enabled/disabled state is the calibration function disabled state, then the zero calibration will not be performed on the initial respiration data.

In some embodiments, the zero calibration algorithm for the initial respiration data may be selected according to the actual needs by those of ordinary skills in the art without any limitation to the present disclosure.

In steps S201 to S208 illustrated in an embodiment of the present disclosure, firstly, the gas flow rate data is determined according to the collection time period and the initial respiration data, and the initial calibration data is selected from the gas flow rate data according to the preset calibration data selection rule and the collection time period. Then, the error evaluation is performed on the initial calibration data according to the preset differential calculation rule to obtain the error evaluation information, and the accumulated duration is determined according to the error evaluation information, the initial calibration data and the collection time period, so as to compare the accumulated duration with the preset calibration duration to obtain duration comparison information. Finally, the zero calibration detection is performed on the initial respiration data according to the duration comparison information to obtain the zero calibration detection information, the zero calibration enabled/disabled state of the initial respiration data is determined according to the zero calibration detection information, and the zero calibration on the initial respiration data is performed according to the zero calibration enabled/disabled state. Therefore, the nebulization control method provided by an embodiment of the present disclosure can perform the zero calibration of the initial respiration data according to the stable state of the gas flow rate data, which reduces the impact of zero offset on the initial respiration data, and improves the accuracy and reliability of the initial respiration data, thereby improving the adaptability of the nebulization device to environmental factor changes.

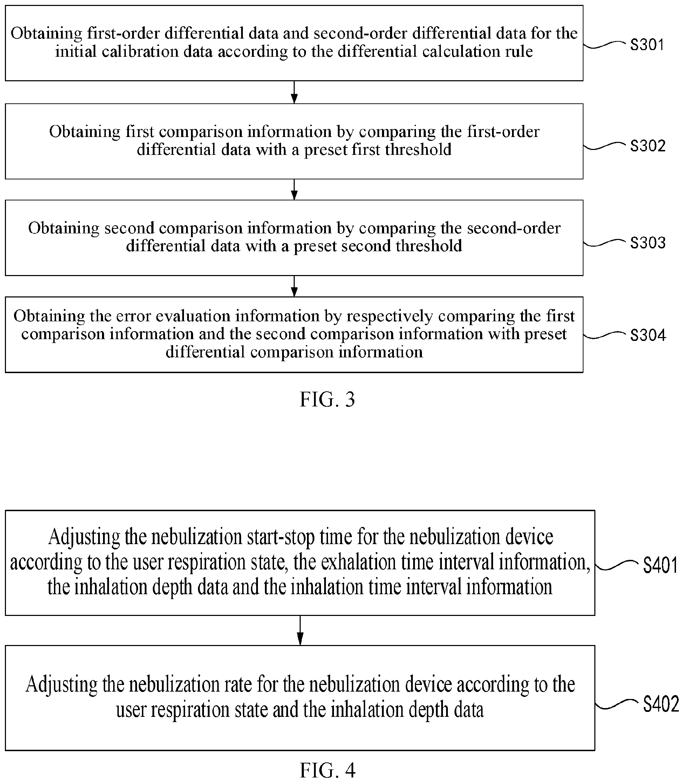

Referring to FIG. 3, in some embodiments, step S203 may include, but is not limited to, steps S301 to S304:

step S301, obtaining first-order differential data and second-order differential data for the initial calibration data according to the differential calculation rule;

step S302, obtaining first comparison information by comparing the first-order differential data with a preset first threshold;

step S303, obtaining second comparison information by comparing the second-order differential data with a preset second threshold; and

step S304, obtaining the error evaluation information by respectively comparing the first comparison information and the second comparison information with preset differential comparison information.

In some embodiments, in step S301, the preset differential calculation rule involves determining the first-order differential data of two adjacent initial calibration data, and determining the second-order differential data for the initial calibration data according to the two adjacent first-order differential data. The first-order differential data is used to describe the change amplitude of the initial calibration data, and the second-order differential data is used to describe the change speed of the initial calibration data.

In some embodiments, in step S302, the first comparison information and the first threshold are used to describe whether the change amplitude of the initial calibration data is stable. If the first comparison information indicates that the first-order differential data is less than or equal to the first threshold, it means that the change amplitude of the initial calibration data is relatively stable. If the first comparison information indicates that the first-order differential data is greater than the first threshold, it means that the change amplitude of the initial calibration data is unstable. The value of the first threshold may be selected according to the actual needs by those of ordinary skills in the art without specific limitation to the present disclosure.

In some embodiments, in step S303, the second comparison information and the second threshold are used to describe whether the change amplitude of the initial calibration data is stable, thereby reflecting whether the initial calibration data is stable. If the second comparison information indicates that the second-order differential data is less than or equal to the second threshold, it means that the change speed of the initial calibration data is small and the change amplitude of the initial calibration data is relatively stable. If the second comparison information indicates that the second-order differential data is greater than the second threshold, it means that the change speed of the initial calibration data is large and the change amplitude of the initial calibration data is unstable. The value of the second threshold may be selected according to the actual needs by those of ordinary skills in the art without specific limitation to the present disclosure. It should be noted that the specific values of the first threshold and the second threshold may be the same or different.

In some embodiments, in step S304, the differential comparison information is used to comprehensively describe the stability of the first-order differential data and the second-order differential data, thereby describing the magnitude of the error of the initial calibration data. If the first comparison information indicates that the first-order differential data is less than or equal to the first threshold, and the second comparison information indicates that the second-order differential data is less than or equal to the second threshold, then the differential comparison information indicates that the error between the initial calibration data is small. That is, the initial calibration data are stable. At this time, the error evaluation information is set to indicate that the initial calibration data pass the error evaluation. If the first comparison information indicates that the first-order differential data is greater than the first threshold, or the second comparison information indicates that the second-order differential data is greater than the second threshold, then the differential comparison information indicates that the error between the initial calibration data is large. That is, the initial calibration data are not stable. At this time, the error evaluation information is set to indicated that the initial calibration data do not pass the error evaluation.

In steps S301 to S304 illustrated in an embodiment of the present disclosure, firstly, the first-order differential data and the second-order differential data of the initial calibration data are obtained according to the differential calculation rule. Then, the first-order differential data is compared to the preset first threshold to obtain the first comparison information, and the second-order differential data is compared to the preset second threshold to obtain the second comparison information. Finally, the first comparison information and the second comparison information are compared to the preset differential comparison information respectively to obtain error evaluation information, which can accurately evaluate whether the initial calibration data are stable, ensuring that the zero calibration is performed on the initial calibration data only when the initial respiration data is stable, and reducing the interference of environmental factors on the accuracy of the zero calibration of the initial respiration data.

In some embodiments, in step S 102, the user respiration parameters may include, but are not limited to: exhalation time interval information, exhalation depth data, inhalation depth data and the inhalation time interval information. The exhalation time interval information is used to describe the duration during which the target user exhales. The inhalation time interval information is used to describe the duration during which the target user inhales. The exhalation depth data is used to describe the maximum exhalation rate of the target user in each exhalation duration. The inhalation depth data is used to describe the maximum inhalation rate of the target user in each inhalation duration. The exhalation time interval information and inhalation depth data are determined according to the zero-crossing point data of the gas flow rate data, the exhalation depth data is determined according to the exhalation time interval information and the gas flow rate data, and the inhalation depth data is determined according to the inhalation time interval information and the gas flow rate data. It should be noted that the gas flow rate data have positive and negative values. Therefore, the gas flow rate data may be first calculated according to the collection time period and the initial respiration data, and then the exhalation time interval information, the exhalation depth data, the inspiratory depth data, and the inhalation time interval information may be calculated according to the gas flow rate data and the collection time period.

In some embodiments, when the target user is in the exhalation state, the gas flow rate data is positive, and the exhalation depth data is the absolute value of the maximum gas flow rate data. When the target user is in the inhalation state, the gas flow rate data is negative, and the inhalation depth data is the absolute value of the minimum gas flow rate data.

In other embodiments, when the target user is in the exhalation state, the gas flow rate data is negative, and the exhalation depth data is the absolute value of the minimum gas flow rate data. When the target user is in the inhalation state, the gas flow rate data is positive, and the inhalation depth data is the absolute value of the maximum gas flow rate data.

In other embodiments, the user respiration parameters may further include: an exhalation cycle and an inhalation cycle. The exhalation cycle is used to describe the time interval between two adjacent exhalation states, and consists of one respiration time interval information and one inhalation time interval information that are adjacent. The inhalation cycle is used to describe the time interval between two adjacent inhalation states, and consists of one inhalation time interval information and one respiration duration that are adjacent.

In some embodiments, in step S103, the user respiration state includes an abnormal respiration state and a stable respiration state. The user respiration state may be determined by performing mutual state detection according to the exhalation time interval information, the exhalation depth data, the inhalation depth data and the inhalation time interval information. Specifically, at least three exhalation time interval information, at least three exhalation depth data, at least three inhalation depth data and at least three inhalation time interval information of the target user are respectively obtained. Then the difference between any two adjacent exhalation time interval information is determined to obtain the exhalation duration difference. The difference between any two adjacent inhalation time interval information is determined to obtain the inhalation duration difference. The difference between any two adjacent exhalation depth data is determined to obtain the exhalation depth difference. The difference between any two adjacent inhalation depth data is determined to obtain the inhalation depth difference. Finally, at least two of the exhalation time interval information, the exhalation depth data, the inhalation depth data, the inhalation time interval information, the exhalation duration difference, the inhalation duration difference, the exhalation depth difference and the inhalation depth difference are selected as the state data to be detected, and compare the state data to be detected to a preset state detection range to determine the abnormal respiration state and the stable respiration state. When the state data to be detected is within the state detection range, the user respiration state is determined to be a stable respiration state. The state detection range may be set to a normal respiratory rate range of a person, usually every 12-20 times/minute. Taking the exhalation depth data and inhalation depth data as an example, if the total number of exhalation depth data per minute is within 12-20 times, and the total number of inhalation depth data per minute is within 12-20 times, then it is determined that the user respiration state is a stable respiration state.

In some embodiments, the abnormal respiration state includes, but is not limited to: a tachypnea state, a bradypnea state, an intermittent respiration state, a Cheyne-Stokes respiration state, a Biot respiration state, a decreased respiration state, an increased respiration state, and a respiratory arrest state. On this basis, the specific abnormal respiration state of the target user may be further determined according to the respiration parameters. Taking the exhalation time interval information and the inhalation time interval information as an example, the number of exhalation cycles and the number of inhalation cycles are first calculated according to the exhalation time interval information and the inhalation time interval information, and then the number of exhalation cycles and the number of inhalation cycles per minute are detected. If the number of exhalation cycles per minute is less than 12, and the number of inhalation cycles per minute is less than 12, then the user respiration state is determined to be a bradypnea state among the abnormal respiration states. If the user respiration state is an abnormal respiration state, prompt information is generated according to the abnormal respiration state to alert the target user of the abnormal respiration state. The prompt information may also include respiration adjustment information which is used to remind the target user how to adjust the abnormal respiration state to a stable respiration state.

In some embodiments, in step S104, the nebulization parameters include: a nebulization start-stop time and a nebulization rate. The nebulization start-stop time is used to control the start or stop of nebulization. The nebulization rate is used to control the speed at which the drug liquid is nebulized.

Referring to FIG. 4, in some embodiments, step S104 may include, but is not limited to, steps S401 to S402:

step S401, adjusting the nebulization start-stop time for the nebulization device according to the user respiration state, the exhalation time interval information, the inhalation depth data and the inhalation time interval information; and

step S402, adjusting the nebulization rate for the nebulization device according to the user respiration state and the inhalation depth data.

In steps S401 to S402 illustrated in an embodiment of the present disclosure, the nebulization start-stop time of the nebulization device is adjusted according to the user respiration state, the exhalation time interval information, the inhalation depth data and inhalation time interval information; and the nebulization rate of the nebulization device is adjusted according to the user respiration state and the inhalation depth data, so that the nebulization device can adjust the nebulization start-stop time and the nebulization rate according to the actual respiration state and the respiration parameters of the target user. This provides a personalized drug nebulization solution to flexibly adapt to the actual respiration condition of the user, improving the accuracy of drug nebulization and reducing drug waste.

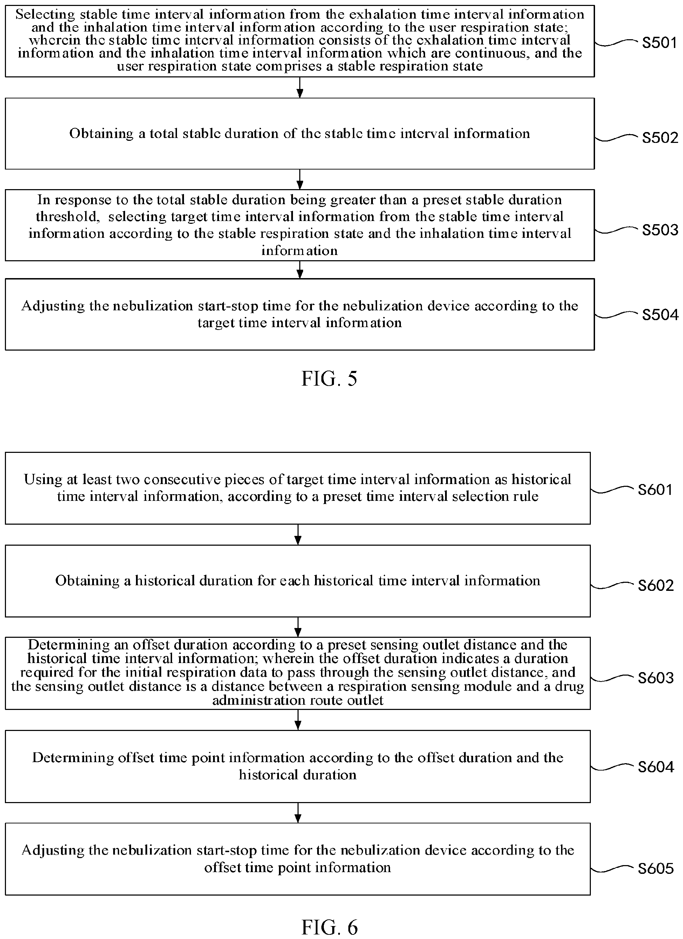

Referring to FIG. 5, in some embodiments, step S401 may include, but is not limited to, steps S501 to S504:

step S501, selecting stable time interval information from the exhalation time interval information and the inhalation time interval information according to the user respiration state; where the stable time interval information consists of the exhalation time interval information and the inhalation time interval information which are continuous, and the user respiration state includes a stable respiration state;

step S502, obtaining the total stable duration of the stable time interval information;

step S503, in a case where the total stable duration is greater than a preset stable duration threshold, selecting target time interval information from the stable time interval information according to the stable respiration state and the inhalation time interval information; and

step S504, adjusting the nebulization start-stop time for the nebulization device according to the target time interval information.

In some embodiments, in step S503, the stable duration threshold is used to describe whether there is a sufficient amount of inhalation time interval information in the stable time interval information for adjusting the nebulization start-stop time for the nebulization device. In a case where the total stable duration is greater than the preset stable duration threshold, which means that the number of the inhalation time interval information in the stable time interval information has met the minimum quantity requirement for adjusting the nebulization start-stop time, then the target time interval information is selected from the stable time interval information according to the stable respiration state and the inhalation time interval information. If the total stable duration is less than the preset stable duration threshold, which means that the number of the inhalation time interval information in the stable time interval information cannot meet the minimum quantity requirement for adjusting the nebulization start-stop time, then jump to step S501 to re-select the stable time interval information from the exhalation time interval information and the inhalation time interval information according to the user respiration state until the total stable duration is greater than the stable duration threshold.

In steps S501 to S504 illustrated in an embodiment of the present disclosure, the stable time interval information is firstly selected from the exhalation time interval information and the inhalation time interval information according to the user respiration state, where the stable time interval information consists of the exhalation time interval information and the inhalation time interval information which are continuous, and the user respiration state includes a stable respiration state. Then, the total stable duration of the stable time interval information is obtained, and the total stable duration is compared with the preset stable duration threshold. In a case where the total stable duration is greater than the preset stable duration threshold, then the target time interval information is selected from the stable time interval information according to the stable respiration state and the inhalation time interval information, and the nebulization start-stop time of the nebulization device is adjusted according to the target time interval information. Therefore, the nebulization control method provided by an embodiment of the present disclosure can obtain sufficient inhalation time interval information from the stable time interval information to accurately synchronize the nebulization start-stop time and the inhalation duration of the target user according to the inhalation time interval information, thereby effectively adjusting the nebulization start-stop time for the nebulization device to ensure the accuracy and reliability of the nebulization start-stop time.

Referring to FIG. 6, in some embodiments, step S504 includes, but is not limited to, steps S601 to S605:

step S601, using at least two consecutive pieces of target time interval information as historical time interval information according to a preset time interval selection rule;

step S602, obtaining a historical duration for each historical time interval information;

step S603, determining an offset duration according to a preset sensing outlet distance and the historical time interval information; where the offset duration indicates a duration required for the initial respiration data to pass through the sensing outlet distance, and the sensing outlet distance is a distance between the respiration sensing module and a drug administration route outlet;

step S604, determining offset time point information based on the offset duration and the historical duration; and

step S605, adjusting the nebulization start-stop time for the nebulization device according to the offset time point information.

In some embodiments, in step S601, the time interval selection rule is used to determine the specific value of the historical time interval information and from which target time interval information to start selecting the historical time interval information. For example, all target period information used is regarded as historical time interval information. Alternatively, four consecutive pieces of target time interval information including the last target time interval information are used as historical time interval information. The time interval selection rule may be set by those of ordinary skills in the art according to actual needs, or may be obtained according to a preset duration selection algorithm without any limitation to the present disclosure.

In some embodiments, in step S603, the drug administration route outlet is the drug output end of the drug administration route. Due to the sensing outlet distance between the respiration sensing module and the drug administration route outlet, there may be a difference between the inhalation time interval information determined according to the initial respiration data and the collection time period and the actual inhalation duration of the drug administration route outlet. Therefore, the offset duration needs to be determined according to the duration required for the drug gas to pass through the sensing outlet distance to compensate for the error between the inhalation time interval information and the actual inhalation duration. Therefore, the nebulization is started in advance according to the offset duration before the target user enters the inhalation duration, so that the drug gas may be obtained in time when the user starts inhalation, and the nebulization is stopped in advance according to the offset duration before the target user enters the exhalation duration to reduce drug waste.

In some embodiments, in step S604, the specific implementation of determining the offset time point information according to the offset duration and the historical duration may be to first determine the average of each historical duration, and then determine the offset time point information according to the average of the historical duration and the offset duration. It may also be possible to input each historical duration and the offset duration into a preset offset time point determination model to output the offset time point information through the offset time point determination model. It may also be other offset time point information determination methods that those of ordinary skills in the art can directly obtain from existing technologies.

In steps S601 to S605 illustrated in an embodiment of the present disclosure, at least two consecutive pieces of target period information are first used as the historical time interval information according to the preset time interval selection rule, and the historical duration of each historical time interval information is obtained. Then, the offset duration is determined according to the preset sensing outlet distance and the historical time interval information, where the offset duration indicates a duration required for the initial respiration data to pass through the sensing outlet distance, and the sensing outlet distance is a distance between the respiration sensing module and the drug administration route outlet. Finally, the offset time point information is determined according to the offset duration and the historical duration, and the nebulization start-stop time of the nebulization device is adjusted according to the offset time point information. Therefore, according to the nebulization control method provided by an embodiment of the present disclosure, the distance between the respiration sensing module and the drug administration route outlet of the nebulization device can be considered, thereby determining the offset duration, and the offset time point information is set according to the historical time interval information and the offset duration, to adjust the nebulization start-stop time, thereby accurately synchronizing the user inhalation duration and the drug output duration, which improves the utilization efficiency of drugs.

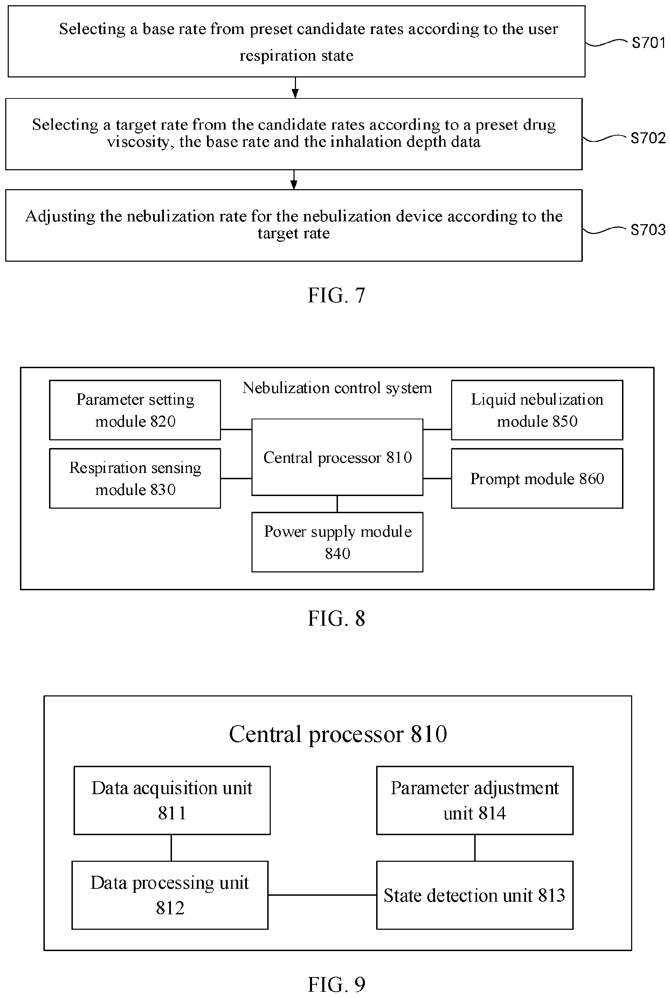

Referring to FIG. 7, in some embodiments, step S402 may include, but is not limited to, steps S701 to S703:

step S701, selecting a base rate from preset candidate rates according to the user respiration state;

step S702, selecting a target rate from the candidate rates according to a preset drug viscosity, the base rate and the inhalation depth data; and

step S703, adjusting the nebulization rate for the nebulization device according to the target rate.

In some embodiments, in step S701, the base rate is used to maintain the nebulization rate of the drugs at the lowest effective level in the exhalation duration to ensure the continuity of drug delivery, and maintain the concentration of the drugs in the nebulization gas at a certain level in the exhalation duration to reduce the time required to nebulize the drugs to the target concentration in the inhalation duration, thereby improving the drug nebulization efficiency of the nebulization device. The specific base rate may be determined according to the user respiration state of the target user. For example, when the user respiration state of the target user is a shallow respiration state, a higher base rate is selected from the preset candidate rates to ensure that even if in a shallow respiration state, a larger dose of drugs may also be delivered to the user to compensate for the reduction in drug inhalation caused by the insufficient respiration capacity of the target user, thereby improving the personalized level of drug nebulization by the nebulization device.

In some embodiments, the base rate may also be selected from the candidate rates according to a preset nebulization mode. For example, for infants or newborns, the base rate corresponding to the newborn mode may be pre-stored in the candidate rates. Therefore, when the nebulization mode is set to the newborn mode, the base rate corresponding to the newborn mode may be automatically matched from the candidate rates to nebulize the drugs at a base rate during the exhalation duration to replenish the drugs for newborns with weak respiration.

In some embodiments, in step S702, the target rate may be selected from the candidate rates according to the preset drug viscosity, the base rate, and the inhalation depth data. Specifically, the initial rate is first calculated according to the preset drug viscosity, the base rate, and the inhalation depth data, and then the target rate is selected from the candidate rates according to the initial rate, where the target rate is closest to the initial rate.

The relationship between drug viscosity, the base rate, the inhalation depth data and the initial rate may be expressed by the following formula:

$$DEP(N) = AT(N) \cdot ATS + ATB;$$

where DEP (N) represents the inhalation depth data of the $N^{th}$ inhalation duration, AT (N) represents the initial rate of the $N^{th}$ inhalation duration, ATS represents the drug viscosity, ATS may usually be determined according to the drug proportion, and ATB represents the base rate. Therefore, after determining the drug viscosity, the base rate, and the inhalation depth data, the initial rate may be determined according to the above formula.

In steps S701 to S703 illustrated in an embodiment of the present disclosure, the base rate is selected from the preset candidate rates according to the user respiration state, and the target rate is selected from the candidate rates according to the preset drug viscosity, the base rate and the inhalation depth data, and finally, the nebulization rate of the nebulization device is adjusted according to the target rate. In this way, the nebulization rate of the nebulization device can be adjusted in real time according to the actual respiration state and the inhalation depth data of the target user, thereby improving the drug utilization efficiency of the nebulization device and the personalized level of the drug nebulization.

Referring to FIG. 8, an embodiment of the present disclosure further provides a nebulization control system, including:
a central processor 810, a parameter setting module 820, a respiration sensing module 830, a power supply module 840, a liquid nebulization module 850 and a prompt module 860.

In some embodiments, the central processor 810 is configured to analyze a respiration state of a target user according to initial respiration data, adjust nebulization parameters according to the respiration state and the initial respiration data, to drive the liquid nebulization module 850 according to the nebulization parameters, and generate prompt information according to an abnormal respiration state.

In some embodiments, an output end of the parameter setting module 820 is electrically connected to an input end of the central processor 810 for setting the nebulization parameters. The method of setting the nebulization parameters may include, but is not limited to, at least one of: touch screen input, key input or wireless signal input, without any limitation to the present disclosure. The set nebulization parameters may include but are not limited to: a drug viscosity, a nebulization start-stop time and a nebulization rate, without specific limitation to the present disclosure.

In some embodiments, an output end of the respiration sensing module 830 is electrically connected to an input end of the central processor 810 for collecting the initial respiration data. The respiration sensing module 830 may include, but is not limited to, at least one of: a temperature sensor, a pressure difference sensor, an infrared photoelectric sensor, a resistive sensor or a capacitive sensor, without any limitation to the present disclosure.

In some embodiments, an output end of the power module 840 is electrically connected to the input end of the central processor 810 for powering the central processor 810.

In some embodiments, an input end of the liquid nebulization module 850 is electrically connected to the output end of the central processor 810, an output end of the liquid nebulization module 850 is connected to an input end of a drug administration route, and an output end of the drug administration route is a drug administration route outlet, where the drug administration route and the drug administration route outlet are not shown in the figure. The liquid nebulization module 850 is used to nebulize drugs according to the nebulization parameters. The liquid nebulization module 850 may use different nebulization drives, such as: mesh nebulization drive, ultrasonic nebulization drive or compression nebulization drive.

In some embodiments, an input end of the prompt module 860 is electrically connected to an output end of the central processor 810 for alerting the target user of the abnormal respiration state according to the prompt information. The prompt module 860 may also be used to give a timing prompt of the total nebulization time. Further, it may be used to prompt the total amount and the remaining amount of the drugs.

Referring to FIG. 9, the central processor 810 provided by an embodiment of the present disclosure may implement the nebulization control method. The central processor 810 includes:

a data acquisition unit 811, configured to acquire initial respiration data of a target user, according to a preset collection time period;

a data processing unit 812, configured to obtain user respiration parameters by performing respiration parameter calculation for the target user according to the collection time period and the initial respiration data;

a state detection unit 813, configured to obtain a user respiration state by performing respiration state detection for the target user according to the user respiration parameters; and

a parameter adjustment unit 814, configured to adjust nebulization parameters of the nebulization device according to the user respiration state and the user respiration parameters.

The specific implementation of the central processor 810 is substantially the same as specific embodiments of the nebulization control method, and will not be described again herein.

An embodiment of the present disclosure further provides an electronic device. The electronic device includes a memory that stores a computer program and a processor that implements the nebulization control method when the processor executes the computer program. The electronic device may be any smart terminal including a tablet computer, a vehicle-mounted computer, and the like.

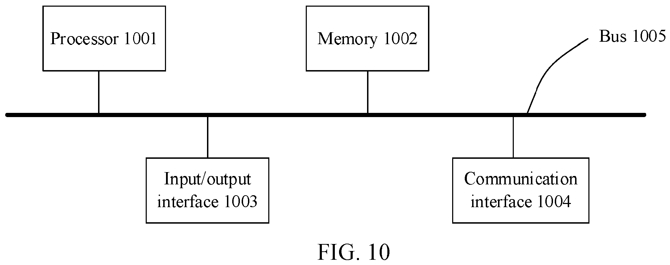

Referring to FIG. 10, which illustrates a hardware structure of an electronic device according to another embodiment. The electronic device includes a processor 1001, a memory 1002, an input/output interface 1003, a communication interface 1004, a bus 1005.

The processor 1001 may be implemented by a general central processor (CPU), a microprocessor, an application specific integrated circuit (ASIC), one or a plurality of integrated circuits or other means for executing relevant programs to implement the technical solutions provided by embodiments of the present disclosure.

The memory 1002 may be implemented in the form of read-only memory (ROM), static storage device, dynamic storage device, or random access memory (RAM). The memory 1002 may store operating systems and other application programs, and when the technical solutions provided by embodiments of the present disclosure are implemented through software or firmware, the relevant program codes are stored in the memory 1002 and called by the processor 1001 to execute the nebulization control method according to the embodiments of the present disclosure.

The input/output interface 1003 is configured to implement information input and output.

The communication interface 1004 is configured to implement communicative internecions between this device and other devices. The communication may be implemented through wired means (such as USB and network cables) or wireless means (such as mobile networks, WIFI, Bluetooth).

The bus 1005 is configured to communicate information between various components (such as the processor 1001, the memory 1002, the input/output interface 1003, and the communication interface 1004) of the device.

The processor 1001, the memory 1002, the input/output interface 1003 and the communication interface 1004 implement communication connections between each other within the device through the bus 1005.

An embodiment of the present disclosure further provides a computer-readable storage medium that stores a computer program which, when executed by a processer, implements the nebulization control method.

As a non-transitory computer-readable storage medium, the memory may be used to store non-transitory software programs and non-transitory computer executable programs. In addition, the memory may include high-speed random access memory, and may also include non-transitory memory, such as at least one magnetic disk storage device, flash memory device, or other non-transitory solid-state storage device. In some embodiments, the memory may optionally include memories located remotely from the processor, and these remote memories may be connected to the processor via a network. Examples of the above-mentioned networks include but are not limited to the Internet, intranets, local area networks, mobile communication networks and combinations thereof.

The nebulization control method and system, and the electronic device are proposed in the present disclosure. Firstly, initial respiration data of a target user is obtained according to a preset collection time period, and respiration parameters of the target user are calculated according to the collection time period and the initial respiration data, to obtain user respiration parameters. Then, a respiration state of the target user is detected according to the user respiration parameters, to obtain a user respiration state. Finally, nebulization parameters of the nebulization device are adjusted according to the user respiration state and the user respiration parameters. Therefore, in the nebulization control method provided by embodiments of the present disclosure, the nebulization parameters of the nebulization device can be adjusted according to the real-time respiration data, so that the user can inhale the nebulized drugs in a specific respiration state, which improves the operating flexibility of the nebulization device, and also reduces the drug waste due to the drug nebulization for a long time, thus improving the utilization of nebulized drugs.

The embodiments of the present disclosure are provided to more clearly illustrate the technical solutions of the present disclosure, and do not constitute a limitation on the technical solutions of the present disclosure. Those of ordinary skills in the art will know that with the evolution of technology and the emergence of new application scenarios, the technical solutions of the present disclosure are equally applicable to similar technical problems.

Those of ordinary skills in the art may understand that the technical solutions illustrated in the figures do not limit the embodiments of the present disclosure, and may include more or fewer steps than those illustrated in the figures, or combine certain steps, or have different steps.

The device embodiments described above are only illustrative, and the units described as separate components may or may not be physically separate, that is, they may be located in one place, or they may be distributed across multiple network units. Part or all of the modules may be selected according to actual needs to implement the objective of the present disclosure.

Those of ordinary skill in the art may understand that all or some steps in the methods, and functional modules in the systems and devices disclosed above may be implemented as software, firmware, hardware, and appropriate combinations thereof.

The terms such as "first", "second", "third", "fourth" (if present) in the description and the accompanying drawings of the present disclosure are used to distinguish similar objects and are not necessarily used to describe specific order or sequence. It should be understood that the data so used is interchangeable under appropriate circumstances so that the embodiments of the present disclosure described herein can be practiced in sequences other than those illustrated or described herein. In addition, the terms such as "include", "comprise" and "have" and any variations thereof are intended to cover non-exclusive inclusions, for example, processes, methods, systems, products, or devices that encompass a series of steps or units does not need to be limited to those steps or units explicitly listed, but may include other steps or units that are not clearly listed or are inherent to those processes, methods, products or devices.

It should be understood that in the present disclosure, the term "at least one" refers to one or more, and the term "a plurality of" refers to two or more. The term "at least one of" or similar expression thereof refers to any combination thereof, including any combination of a single item or a plurality of items. For example, at least one of a, b or c may include: a, b, c, "a and b", "a and c", "b and c", or "a and b and c", where a, b, c may be singular or plural.

In the several embodiments provided in the present disclosure, it should be understood that the disclosed systems and methods may be implemented via other means. For example, the system embodiments described above are only illustrative. For example, the division of the above modules is only a logical function division. In actual implementation, there may be other division methods. For example, a plurality of modules or assemblies may be combined or may be integrated into another system, or some features may be ignored, or not executed. On the other hand, the coupling or direct coupling or communication connection between the systems or units shown or discussed may be indirect coupling or communication connection through some interfaces, which may be in electrical, mechanical or other forms.

The modules described above as separate components may or may not be physically separated. The components shown as modules may or may not be physical modules, that is, they may be located in one place, or they may be distributed to multiple network units. Part or all of the modules may be selected according to actual needs to implement the objective of the present disclosure.

In addition, each functional module in each embodiment of the present disclosure may be integrated into a processing unit, or each module may exist physically alone, or two or more modules may be integrated into one module. The above integrated modules may be implemented in the form of hardware or software function modules.

Integrated modules may be stored in a computer-readable storage medium if they are implemented in the form of software function modules and sold or used as independent products. Therefore, the technical solutions of the present disclosure or the part that contributes to the existing technologies or all or part of the technical solution may substantially be embodied in the form of a software product. The computer software product is stored in a storage medium, including multiple instructions for causing a computer device (which may be a personal computer, a server, or a network device, or the like) to execute all or part of the steps of the methods of each embodiment of the present disclosure. The aforementioned storage medium includes: a U disk, a mobile hard disk, a read-only memory (ROM), a random access memory (RAM), a magnetic disk or an optical disk and other medium that may store programs.

The preferred embodiments of the present disclosure have been described above with reference to the accompanying drawings, but they are not intended to limit the scope of the present disclosure. Any modifications, equivalent substitutions and improvements made by those of ordinary skills in the art without departing from the scope and essence of the present disclosure shall be within the scope of the present disclosure.

## Claims

1.  A nebulization control method, applied to a nebulization device, the method comprising:

    obtaining (S101) initial respiration data of a target user according to a collection time period;
    obtaining (5102) user respiration parameters by performing respiration parameter calculation for the target user according to the collection time period and the initial respiration data;
    obtaining (S103) a user respiration state by performing respiration state detection for the target user according to the user respiration parameters; and
    adjusting (S104) nebulization parameters for the nebulization device according to the user respiration state and the user respiration parameters.

2.  A method according to claim 1, wherein the user respiration parameters comprise exhalation time interval information, inhalation depth data and inhalation time interval information; the nebulization parameters comprise a nebulization start-stop time and a nebulization rate; wherein the step of adjusting (S104) nebulization parameters for the nebulization device according to the user respiration state and the user respiration parameters comprises:

    adjusting (S401) the nebulization start-stop time for the nebulization device according to the user respiration state, the exhalation time interval information, the inhalation depth data and the inhalation time interval information; and
    adjusting (S402) the nebulization rate for the nebulization device according to the user respiration state and the inhalation depth data.

3.  A method according to claim 2, wherein the step of adjusting (S401) the nebulization start-stop time for the nebulization device according to the user respiration state, the exhalation time interval information, the inhalation depth data and the inhalation time interval information comprises:

selecting (S501) stable time interval information from the exhalation time interval information and the inhalation time interval information according to the user respiration state; wherein the stable time interval information consists of the exhalation time interval information and the inhalation time interval information which are continuous, and the user respiration state comprises a stable respiration state;

obtaining (S502) a total stable duration of the stable time interval information;

in response to the total stable duration being greater than a preset stable duration threshold, selecting (S503) target time interval information from the stable time interval information according to the stable respiration state and the inhalation time interval information; and

adjusting (S504) the nebulization start-stop time for the nebulization device according to the target time interval information.

4. A method according to claim 3, wherein the step of adjusting (S504) the nebulization start-stop time for the nebulization device according to the target time interval information comprises:

using (S601) at least two consecutive pieces of target time interval information as historical time interval information, according to a preset time interval selection rule;

obtaining (S602) a historical duration for each historical time interval information;

determining (S603) an offset duration according to a preset sensing outlet distance and the historical time interval information; wherein the offset duration indicates a duration required for the initial respiration data to pass through the sensing outlet distance, and the sensing outlet distance is a distance between a respiration sensing module and a drug administration route outlet;

determining (S604) offset time point information according to the offset duration and the historical duration; and

adjusting (S605) the nebulization start-stop time for the nebulization device according to the offset time point information.

5. A method of claim 2, wherein the step of adjusting (S402) the nebulization rate for the nebulization device according to the user respiration state and the inhalation depth data comprises:

selecting (S701) a base rate from preset candidate rates according to the user respiration state;

selecting (S702) a target rate from the candidate rates according to a preset drug viscosity, the base rate and the inhalation depth data; and

adjusting (S703) the nebulization rate for the nebulization device according to the target rate.

6. A method according to claim 1, wherein after obtaining (S101) initial respiration data of a target user according to a collection time period, the method further comprises:

determining (S201) gas flow rate data according to the collection time period and the initial respiration data;

selecting (S202) initial calibration data from the gas flow rate data according to a preset calibration data selection rule and the collection time period;

obtaining (S203) error evaluation information by performing error evaluation on the initial calibration data according to a preset differential calculation rule;

determining (S204) an accumulated duration according to the error evaluation information, the initial calibration data and the collection time period;

obtaining (S205) duration comparison information by comparing the accumulated duration and a preset calibration duration;

obtaining (S206) zero calibration detection information by performing zero calibration detection on the initial respiration data according to the duration comparison information;

determining (S207) zero calibration enabled/disabled state of the initial respiration data according to the zero calibration detection information; and

performing (S208) zero calibration on the initial respiration data according to the zero calibration enabled/disabled state.

7. A method according to claim 6, wherein the step of obtaining (S203) error evaluation information by performing error evaluation on the initial calibration data according to the preset differential calculation rule comprises:

obtaining (S301) first-order differential data and second-order differential data for the initial calibration data according to the differential calculation rule;

obtaining (S302) first comparison information by comparing the first-order differential data with a preset first threshold;

obtaining (S303) second comparison information by comparing the second-order differential data with a preset second threshold; and

obtaining (S304) the error evaluation information by comparing the first comparison information and the second comparison information with preset differential comparison information respectively.

8.  A nebulization control system, for implementing the nebulization control method according to any one of claims 1 to 7, the nebulization control system comprising a central processor (810), the central processor (810) comprising:

    a data acquisition unit (811), configured to acquire initial respiration data of a target user according to a collection time period;
    a data processing unit (812), configured to obtain user respiration parameters by performing respiration parameter calculation for the target user according to the collection time period and the initial respiration data;
    a state detection unit (813), configured to obtain a user respiration state by performing respiration state detection for the target user according to the user respiration parameters; and
    a parameter adjustment unit (814), configured to adjust nebulization parameters of the nebulization device according to the user respiration state and the user respiration parameters.

9.  A nebulization control system according to claim 8, further comprising: a power supply module (840), a parameter setting module (820), a liquid nebulization module (850), a respiration sensing module (830) and a prompt module (860), wherein,

    an output end of the parameter setting module (820) is electrically connected to an input end of the central processor (810), to set the nebulization parameters;
    an output end of the respiration sensing module (830) is electrically connected to the input end of the central processor (810), to collect the initial respiration data;
    an output end of the power supply module (840) is electrically connected to the input end of the central processor (810), to supply power to the central processor (810);
    an input end of the liquid nebulization module (850) is electrically connected to an output end of the central processor (810), and an output end of the liquid nebulization module (850) is connected to an input end of a drug administration route, to perform drug nebulization according to the nebulization parameters; and
    an input end of the prompt module (860) is electrically connected to the output terminal of the central processor (810), to prompt the user respiration state according to prompt information.

10. An electronic device, comprising a memory (1002) and a processor (1001), where the memory (1002) stores a computer program which, when executed by the processor (1001), implements a nebulization control method according to any one of claims 1 to 7.

| Obtaining initial respiration data of a target user according to a preset collection time period | S101 |

$\downarrow$

| Obtaining user respiration parameters by performing respiration parameter calculation for the target user according to the collection time period and the initial respiration data | S102 |

$\downarrow$

| Obtaining a user respiration state by performing respiration state detection for the target user according to the user respiration parameters | S103 |

$\downarrow$

| Adjusting nebulization parameters for the nebulization device according to the user respiration state and the user respiration parameters | S104 |

FIG. 1

Determining gas flow rate data according to the collection time period and the initial respiration data ⟋ S201

Selecting initial calibration data from the gas flow rate data according to a preset calibration data selection rule and the collection time period ⟋ S202

Obtaining error evaluation information by performing error evaluation on the initial calibration data according to a preset differential calculation rule ⟋ S203

Determining an accumulated duration according to the error evaluation information, the initial calibration data and the collection time period ⟋ S204

Obtaining duration comparison information by comparing the accumulated duration and a preset calibration duration ⟋ S205

Obtaining zero calibration detection information by performing zero calibration detection on the initial respiration data according to the duration comparison information ⟋ S206

Determining zero calibration enabled/disabled state of the initial respiration data according to the zero calibration detection information ⟋ S207

Performing zero calibration on the initial respiration data according to the zero calibration enabled/disabled state ⟋ S208

FIG. 2

| Obtaining first-order differential data and second-order differential data for the initial calibration data according to the differential calculation rule | S301 |
|---|---|

| Obtaining first comparison information by comparing the first-order differential data with a preset first threshold | S302 |
|---|---|

| Obtaining second comparison information by comparing the second-order differential data with a preset second threshold | S303 |
|---|---|

| Obtaining the error evaluation information by respectively comparing the first comparison information and the second comparison information with preset differential comparison information | S304 |
|---|---|

FIG. 3

| Adjusting the nebulization start-stop time for the nebulization device according to the user respiration state, the exhalation time interval information, the inhalation depth data and the inhalation time interval information | S401 |
|---|---|

| Adjusting the nebulization rate for the nebulization device according to the user respiration state and the inhalation depth data | S402 |
|---|---|

FIG. 4

Selecting stable time interval information from the exhalation time interval information and the inhalation time interval information according to the user respiration state; wherein the stable time interval information consists of the exhalation time interval information and the inhalation time interval information which are continuous, and the user respiration state comprises a stable respiration state

S501

Obtaining a total stable duration of the stable time interval information

S502

In response to the total stable duration being greater than a preset stable duration threshold, selecting target time interval information from the stable time interval information according to the stable respiration state and the inhalation time interval information

S503

Adjusting the nebulization start-stop time for the nebulization device according to the target time interval information

S504

FIG. 5

Using at least two consecutive pieces of target time interval information as historical time interval information, according to a preset time interval selection rule

S601

Obtaining a historical duration for each historical time interval information

S602

Determining an offset duration according to a preset sensing outlet distance and the historical time interval information; wherein the offset duration indicates a duration required for the initial respiration data to pass through the sensing outlet distance, and the sensing outlet distance is a distance between a respiration sensing module and a drug administration route outlet

S603

Determining offset time point information according to the offset duration and the historical duration

S604

Adjusting the nebulization start-stop time for the nebulization device according to the offset time point information

S605

FIG. 6

Selecting a base rate from preset candidate rates according to the user respiration state — S701

Selecting a target rate from the candidate rates according to a preset drug viscosity, the base rate and the inhalation depth data — S702

Adjusting the nebulization rate for the nebulization device according to the target rate — S703

FIG. 7

Nebulization control system

Parameter setting module 820

Respiration sensing module 830

Central processor 810

Liquid nebulization module 850

Prompt module 860

Power supply module 840

FIG. 8

Central processor 810

Data acquisition unit 811

Parameter adjustment unit 814

Data processing unit 812

State detection unit 813

FIG. 9

FIG. 10

## EUROPEAN SEARCH REPORT

**Application Number**

EP 24 22 2915

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2007/157931 A1 (PARKER RICHARD [US] ET AL) 12 July 2007 (2007-07-12) | 1-3,5, 8-10 | INV. G16H20/40 G16H40/40 |
| Y | * abstract * * paragraphs [0013], [0014], [0042], [0073], [0074], [0077], [0078], [0082] - [0084], [0109], [0110], [0130] - [0132] * | 4,6,7 | |
| X | US 2007/125370 A1 (DENYER JONATHAN S H [GB] ET AL) 7 June 2007 (2007-06-07) | 1-3,5, 8-10 | |
| Y | * abstract * * paragraphs [0008] - [0012], [0051], [0067] - [0070] * | 4,6,7 | |
| X | EP 4 424 233 A1 (FEELLIFE HEALTH INC [CN]) 4 September 2024 (2024-09-04) | 1-3,5, 8-10 | |
| Y | * abstract * * paragraphs [0004], [0005], [0015], [0061] * | 4,6,7 | |
| Y | US 2022/339392 A1 (SEGAL MICHAEL [US] ET AL) 27 October 2022 (2022-10-27) * paragraphs [0011], [0060] - [0062] * | 4 | **TECHNICAL FIELDS SEARCHED (IPC)** G16H |
| Y | CN 118 576 180 A (INST BIOMEDICAL ENG CAMS) 3 September 2024 (2024-09-03) * abstract * * page 8, paragraph 5 - page 9, paragraph 1 * * page 11, paragraph 3 * | 6,7 | |
| Y | WO 00/64345 A1 (BIRD PRODUCTS CORP [US]) 2 November 2000 (2000-11-02) * the whole document * | 6,7 | |

The present search report has been drawn up for all claims

| Place of search | Date of completion of the search | Examiner |
|---|---|---|
| Munich | 7 June 2025 | Thumb, Werner |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or after the filing date
D : document cited in the application
L : document cited for other reasons

.......................................................................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 22 2915

This annex lists the patent family members relating to the patent documents cited in the above-mentioned European search report.
The members are as contained in the European Patent Office EDP file on
The European Patent Office is in no way liable for these particulars which are merely given for the purpose of information.

07-06-2025

| Patent document cited in search report | | Publication date | Patent family member(s) | | Publication date |
|---|---|---|---|---|---|
| US 2007157931 | A1 | 12-07-2007 | US | 2007157931 A1 | 12-07-2007 |
| | | | WO | 2007008825 A2 | 18-01-2007 |
| US 2007125370 | A1 | 07-06-2007 | AU | 2003286259 A1 | 15-06-2004 |
| | | | BR | 0315862 A | 20-09-2005 |
| | | | CA | 2502728 A1 | 03-06-2004 |
| | | | EP | 1565223 A1 | 24-08-2005 |
| | | | GB | 2396825 A | 07-07-2004 |
| | | | JP | 4639083 B2 | 23-02-2011 |
| | | | JP | 2006506151 A | 23-02-2006 |
| | | | US | 2007125370 A1 | 07-06-2007 |
| | | | WO | 2004045690 A1 | 03-06-2004 |
| EP 4424233 | A1 | 04-09-2024 | CN | 116212177 A | 06-06-2023 |
| | | | EP | 4424233 A1 | 04-09-2024 |
| | | | PT | 4424233 T | 22-05-2025 |
| US 2022339392 | A1 | 27-10-2022 | US | 2020330719 A1 | 22-10-2020 |
| | | | US | 2022339392 A1 | 27-10-2022 |
| CN 118576180 | A | 03-09-2024 | NONE | | |
| WO 0064345 | A1 | 02-11-2000 | AU | 4250700 A | 10-11-2000 |
| | | | US | 6135967 A | 24-10-2000 |
| | | | WO | 0064345 A1 | 02-11-2000 |

EPO FORM P0459

For more details about this annex : see Official Journal of the European Patent Office, No. 12/82